# EUROPÄISCHE PATENTANMELDUNG

(11) **EP 3 607 903 A1**
(43) Veröffentlichungstag der Anmeldung: **12.02.2020**
(21) Anmeldenummer: 18000666.0
(22) Anmeldetag: 09.08.2018
(51) Int. Cl.: A61B 17/50, A46B 9/02, A46B 9/12, A46B 13/02, A46B 13/08

(54) **VORRICHTUNG ZUR SANFTEN ZECKENENTFERNUNG DURCH ROTATION**

(71) Anmelder: Stehr, Horst, 95447 Bayreuth (DE)
(72) Erfinder: Stehr, Horst, 95447 Bayreuth (DE)
(74) Vertreter: Viering, Jentschura & Partner mbB Patent- und Rechtsanwälte

(57) **Zusammenfassung**

Der *Tick-Rotator* ermöglicht bei Mensch und Tier und gerade bei kleinen Kindern jeden Alters ein angstfreies, unkompliziertes und sanftes Entfernen einer Zecke aus der Haut.
Nur ein leichtes "Kitzeln" ist auf der Haut zu spüren. Auch bei mehrfachen Versuchen ist ein Abriss des Zeckenkörpers noch nicht vorgekommen.
Auch für den gewerblichen Gebrauch ist der *Tick-Rotator* geeignet, bevor man versucht, die Zecke mit einem Greif- oder Hebelinstrument gegen ihren Willen herauszuziehen.
Hier ist eine gewerbliche Version aus Edelstahl, die dann z.B. auch mit Netzbetrieb elektrisch arbeitet, denkbar.
Der *Tick-Rotator* ist klein, handlich, transportabel und kann von jedem überall angewendet werden.
Vor der Benutzung ist ein Funktionstest in Bezug auf den Ladezustand der Energieträger notwendig.
Nach der Benutzung sollte der Pinselaufsatz gereinigt, desinfiziert oder bei Notwenigkeit ausgetauscht werden.
Ebenso sollte die Wunde bzw. die Einstichstelle nach einer Benutzung mit Desinfektionsspray desinfiziert werden.

Durch die Benutzung des *Tick-Rotator's* kann viel Leid und Angst vermieden werden.
Ein schnelles, unkompliziertes, sicheres und komplettes Entfernen auch der kleinsten Zecke nach einem Stich ist von bedeutender Wichtigkeit in Bezug auf übertragbare Krankheitserreger und risikofrei mit dem *Tick-Rotator* umsetzbar!

## Beschreibung

Der *Tick-Rotator* (Zecken-Dreher) basiert auf der sanften Entfernung einer bereits zugestochenen Zecke aus der Haut ihres Wirtes.

Hierbei wird die Zecke **nicht** mit einem Greifmechanismus wie Pinzette, Schlinge, Karte oder Zange gegriffen, sondern in einen, speziell für Zecken zugeschnittenen Pinsel eingebettet, durch eine sanfte kreisende Rotation drehend stimuliert und zum **Selbstloslassen** bewegt.

Die Zecke wird durch elektrische, gleichmäßige und langsame Pinselrotationsintervalle in eine für sie "unbequeme" Situation gebracht, sodass sie ihren verankerten Stechapparat löst um nach einer anderen "angenehmeren" Einstichstelle am Wirt zu suchen.

Somit kommt es für die Zecke bei der Entfernung mit dem *Tick-Rotator* zu keiner Panik-, Todesangst- oder Stresssituation wie z.B. durch Quetschung, Greifen oder Körperteilabtrennung mit einer Pinzette oder Zange und einer damit eventuell verbundenen Gefahr der bakteriellen Übertragung (z.B. Borreliose).

Zudem sei noch erwähnt, dass es auch bei der Entfernung einer Zecke durch einen Arzt mit der feinsten medizinischen Pinzette immer wieder zu einem Zeckenabriss kommen kann. Gerade die kleineren Zecken (Larven oder Nymphen) vor ihrer Häutung haben so winzig filigrane Stechwerkzeuge, dass ein perfektes Greifen nahezu unmöglich ist oder weil sich der Patient, zum Beispiel ein Kind, nicht lange genug ruhig verhält. Hier ist leider auch die feinste medizinische Spezialpinzette noch zu groß.

Dem Arzt bleibt bei einem Abriss meist nur die partielle chirurgische Entfernung des Rest-Stechapparates der Zecke übrig.

### Mit dem Tick-Rotator kann das nicht passieren!

Genau hierfür gibt es den *Tick-Rotator.* Er ist extra speziell genau so konstruiert und vorne am Pinsel so geschnitten, dass er auch die kleinste Larve komplett entfernt.

### Gebrauchsanleitung:

Vor Gebrauch wird der Energieträger/ Batterie in den *Tick-Rotator* eingesetzt. Danach wird vorne die Zentrierhülse abgenommen, um den gewählten Pinselaufsatz in die Aufnahmevorrichtung zu stecken.

Daraufhin wird die Zentrierhülse wieder montiert.

Durch das leichte Aufsetzen des Pinsels und der Hülse mit dem Zeckenkörper in der dafür vorgesehenen Vertiefung in der Mitte des Pinsels verhängt sich die bereits gestochene Zecke mit ihren Beinen und ihrem Körper zwangsläufig in den einzelnen Pinselhärchen.

Wird nun die erste Rotation durch Drücken des Schalters am *Tick-Rotator* nach links oder rechts gestartet, dreht sich die Zecke mit. Jedoch dreht sich die Zecke nicht bei jeder Pinselumdrehung mit Deshalb braucht man bei Bedarf bis zu vier Rotationsintervalle.

Je nach Größe der Zecke und ihrem angeborenen Greifreflex löst sie sich nach spätestens fünf Umdrehungen um ihre eigene Achse aus der Einstichstelle und sucht sich unversehrt und lebendig eine neue.

Bei einer Larve (0,1mm bis 0,8mm) oder Nymphe (0,8mm bis 2,0mm) kann es aufgrund ihrer geringen Körpergröße bis zu vier Intervallen sanfter gleichmäßiger Rotation des Pinsels bedürfen bis sie schließlich loslässt, da die winzigen Zeckenbeine und der Körper schwerer zu drehen sind.

Hierbei ist es wichtig, dass jedes Rotationsintervall mit Zirkulation des Pinsels immer kontinuierlich und ohne Ruckeln und Druck stattfindet. Es darf kein Druck auf die Zecke bzw. auf die Haut ausgeübt werden. Ein leichtes Aufsetzen des Pinsels auf die Haut bzw. der Zecke reicht aus. Hierzu dient die durchsichtige Zentrierhülse als Unterstützung.

Jede Anwendung wird in Intervallen durchgeführt.

Jedes Benutzerintervall, auch das Startintervall zu Beginn dauert ca. 10 Sekunden.

Je nach Zeckengröße und Körpereinstichstelle kann der Benutzer die Intervalle beliebig wiederholen.

Zwischen den Intervallen nutzt der Anwender die Unterbrechung für eine vorsichtige Sichtung und Überprüfung der Zecke durch anheben der Zentrierhülse und des Pinsels, ob sie schon losgelassen hat oder nicht. Sollte die Zecke noch festsitzen, wird das nächste 10-Sekundeintervall durchgeführt.

Bei Larven hat sich manchmal gezeigt, dass bis zu vier Intervalle notwendig sind.

Für größere Zecken, die sich bereits auf einen Körperdurchmesser von ca. 5mm und mehr vollgesaugt haben ist der *Tick-Rotator* nicht zu empfehlen, da die Zecke dann so groß geworden ist, dass ihr Körper mit dem Pinsel nicht mehr im Sinne der Beschreibung erfasst werden kann. Die Intervalle mit ihren sanften langsamen Rotationen und den kurzen Pausen assoziieren der Zecke eine natürliche, nicht belastende Beeinflussung, die sie auch aus der Natur durch kratzen ihres Wirtes oder durch am Wirt streifende Äste oder Rinde kennt.

### Die Zecke und bereits patentierte Vorrichtungen:

Nur durch den vorsichtigen Zug der Zecke nach oben, den Widerhaken der Zecke entgegengesetzt, mit einem Greifwerkzeug (z.B. Pinzette, Schlinge, Karte oder Zange) ist es ein riskantes "Kräftezugspiel" zwischen Zecke und dem Entferner. Auch das Verfahren einer Zeckenschlinge basiert auf dem Ergreifen der Zecke und einem anschließenden riskanten Zug.

Ein Entfernen ist somit immer risikobehaftet.

Greift ein Werkzeug minimalst (1/10 Millimeter) zu hoch, wird die Zecke gequetscht oder/ und abgerissen.

Greift es zu tief, wird der Patient schmerzhaft gezwickt und die bereits gereizte, gestochene Hautoberfläche wird zusätzlich unnötig belastet. Bei den kleinen Larven und Nymphen, noch dazu an schwer zugänglichen Körperstellen, ist es nahezu unmöglich ein sicheres Entfernen geschweige denn sicheres Greifen zu gewährleisten. Larven sind selbst mit bloßem Auge kaum erkennbar. Hier ist eine Rotation mit Greifmechanismen quasi unmöglich.

Beim *Tick-Rotator* ist das Abriss-Risiko so nicht vorhanden.

### Anmerkung:

Etwa 80% aller Zeckenstiche beim Menschen werden von kleinen Larven oder Nymphen getätigt.

Eine ausgewachsene größere Zecke wird oft schon beim Krabbeln oder beim Abtasten der Haut bemerkt und es kommt somit im Vergleich seltener zum Stich oder gar zum Saugen.

Ab dieser Größe schrumpft zwar das Risiko des Abreißens der Zecke mit einem Greifwerkzeug gegenüber der Nymphe, bleibt aber auch hier dennoch bestehen!

Alle bisher patentierten Vorrichtungen müssen die Zecke zuerst greifen und dann entfernen. Keine dieser Patente kann eine Larve der Größe von 0,1mm erfassen.

Das widerspenstige Stechwerkzeug jeder Zecke bleibt dann in der Haut verankert stecken und kann eine unangenehme langwierige Entzündung nach sich ziehen, bis es nach langer Ausheilungsphase aus der Haut herauswächst und die Wunde dann endlich heilen kann.

Es wird oft diskutiert, ob ein Drehen der Zecke nach links oder nach rechts zum Entfernen aus der Haut förderlich oder überflüssig ist. Hierbei ist festzuhalten, dass eine Zecke kein Gewinde hat oder sich nicht kreisförmig, wie eine Schraube, in die Haut des Wirtes bohrt. Ferner sticht eine Zecke in die Haut und "sägt" ihre beiden parallel angeordneten "Stechgraber" abwechselnd geradlinig in die Haut.

Die seitlich daran angeordneten winzigen Widerhaken sorgen u.a. für den festen Halt der Zecke.

Einzigartig und nahezu perfekt hat die Natur die Zecke mit ihrem Stechmechanismus und dessen Funktion ausgestattet.

Dennoch muss die Zecke diesen Mechanismus bzw. ihre Verankerung in der Haut lösen können, da sie bis zu ihrer geschlechtlichen Ausreifung zur "erwachsenen" Zecke vorher zwei Entwicklungsstadien durchlaufen muss.

Sie wird als Larve geboren, wird dann zur Nymphe und schließlich zur männlichen oder weiblichen Zecke.

Zwischen jeder dieser Stadien häutet sie sich und braucht jedes Mal unbedingt wieder eine neue Einstichstelle.

Den ersten Wirt muss Sie nach kurzer Zeit aber loslassen und sich häuten.

Danach muss sie sich als Nymphe erneut einen Warmblütler für den nächsten Stich suchen.

Auch hiernach muss die Nymphe wieder loslassen, um dann, nach ihrer letzten Häutung, zur ausgewachsenen geschlechtsreifen Zecke zu werden. Erst jetzt setzt sie zum letzten Stich an um sich voll Blut zu saugen.

Folglich muss eine Zecke also bis zu ihrer Ausreifung dreimal ihren Wirt bzw. ihre Eistichstelle wechseln und somit bis zum Ende auch dreimal loslassen können!

Ungeklärt ist, ob sich eine Zecke während der Zeit in der Haut des Wirtes dreht oder sie ihren Stechapparat beim Verlassen des Wirtes drehend aus der Haut zieht. Durch das jedoch dauerhafte, langsame und sanfte Drehen der Zecke mit dem *Tick-Rotator*, mehrfach um ihre eigene Achse löst sie unversehrt ihren Stechapparat in Sekundenschnelle aus der Haut ihres Wirtes und krabbelt direkt unversehrt weiter.

### Patentanmeldung für den Tick-Rotator:

Als Patent anzumelden ist die beschriebene Gesamtkonstruktion *Tick-Rotator* mit zeckenspezifischen Pinselaufsatz und seiner Funktionsweise.

In Verbindung sind die drei Pinselaufsätze mit ihrer Schnittform und Beschaffenheit auch als geräteunabhängiger Aufsatz als Patent anzumelden.

### Technischer Aufbau des Tick-Rotator's:

Der *Tick-Rotator* hat eine handliche, zylindrische Stiftform ähnlich wie eine kleine Stabtaschenlampe mit einer beidseitigen Vertiefung im Gehäuse, um ihn ähnlich wie einen Füllfederhalter griffsicher und präzise bedienen zu können.
Länge gesamt: ca.12 bis 15cm
Durchmesser Gehäuse: ca. 1,5 bis 3cm

Er wird hauptsächlich elektrisch betrieben, um für jeden Benutzer immer dasselbe, gleichmäßige, langsame und dauerhafte Rotationsintervall gewährleisten zu können. Eine ausgefeilte rein mechanische Version wäre denkbar, gewährleistet aber nur schwer eine für den Laien immer wieder gleiche Benutzung.

Diesbezüglich setzt sich der elektrische *Tick-Rotator* aus sechs nacheinander zusammengebauten Bauelementen zusammen:
1 Pinselaufsätze
2 Aufnahmevorrichtung für Pinselaufsätze
3 Elektromotor mit Getriebe und Antriebswelle
4 Gehäuse mit Batterie und Verkabelung
5 Gehäusedeckel mit Schalter
6 Zentrierhülse

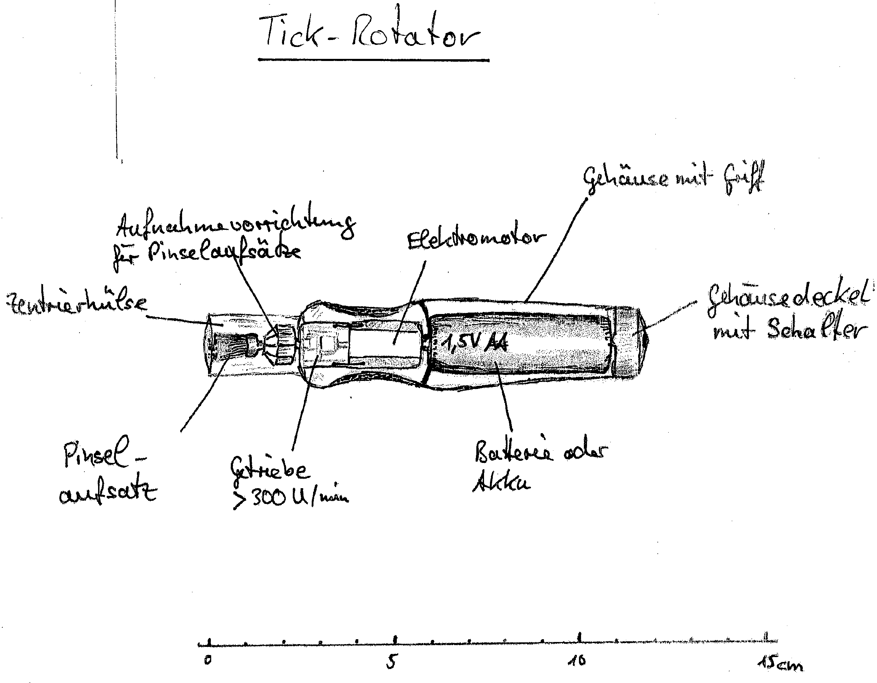

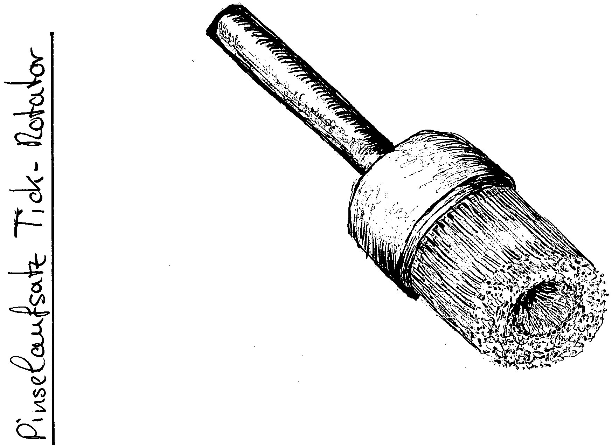

## Patentansprüche

E 1 Pinselaufsätze : Es gibt drei Aufsatzgrößen mit jeweils verschiedengroß geschnittenen Vertiefungen und Zuschnitten. Larven, Nymphen und Zecken. Für jedes Entwicklungsstadium der Zecke einen passenden Aufsatz.
1.1. Aufsatzpinsel **klein für Larven** (ca. 0.1mm bis 0,8mm):
Pinseldurchmesser: ca.5mm
Pinselhaarlänge Randhaare: ca.6mm
Kegeldurchmesser: ca.0,1 bis 0,8mm
Kegelhöhe: ca.1,5mm
1.2. Aufsatzpinsel **mittel für Nymphen** (ca. 0,8mm bis 2mm):
Pinseldurchmesser: ca.5mm
Pinselhaarlänge Randhaare: ca.6mm
Kegeldurchmesser: ca.0,8 bis 2mm
Kegelhöhe: ca. 2,5mm
1.3. Aufsatzpinsel **groß für geschlechtsreife Zecken** (ca. 2mm bis 3,5mm):
Pinseldurchmesser: ca.7mm
Pinselhaarlänge Randhaare: ca.6mm
Kegeldurchmesser: ca.2mm bis 3,0mm
Kegelhöhe: ca. 3,5mm
Jeder Pinselaufsatz hat eine runde, dichte Haaranordnung (Rundpinsel).
Die Pinselhaare bestehen aus weichen Kunst- oder Naturhaaren wie z.B. Ziegenhaare.
Keine Borsten, da die Zecke nicht unnötig belastet oder gereizt werden darf. Der Pinsel hat je nach Größe eine dementsprechend nach innen kegelförmig geschnittene, kleine Vertiefung an der geraden Pinselauflagefläche.
Diese Kegelvertiefung dient dazu, sie über den Zeckenkörper zu platzieren ohne diesen zu quetschen, sodass sich die Zecke mit dem Pinsel dreht.
Der Pinsel hat an seiner Rückseite einen Aufnahmestift aus Metall oder
Kunststoff um ihn in der Aufnahmevorrichtung zu fixieren.

**2.** Aufnahmevorrichtung für Pinselaufsätze:
Vorne am *Tick-Rotator* befindet sich die Aufnahmevorrichtung für die drei variabel fixierbaren Pinselaufsätze.
Diese Aufnahmevorrichtung kann z.B. ein kleines Bohrfutter, eine Steckklemme oder ähnliches aus Metall oder Kunststoff sein und ist auf der Antriebswelle fest verbaut.
Sie dient dazu die Pinselaufsätze festzuhalten. Durch einfaches Auf- und Zudrehen kann der Pinselaufsatz bei Bedarf je nach Zeckengröße problemlos gewechselt werden.
Ein Dreh- oder ein Steckmechanismus kommt hier infrage.

**3.** Elektromotor mit Getriebe und Antriebswelle:
Der Elektromotor treibt das Getriebe mit seiner Antriebswelle an.
Dieser arbeitet mit 1,5 Volt mit etwa 10 mNm und erzeugt, reduziert durch das Getriebe, am Ende etwa 60 bis max. 150 Umdrehungen pro Minute.
Die gleichmäßige Umdrehungszahl darf nicht zu hoch sein, da zum einen die Zecke zu schnell gedreht werden kann und zum anderen eine unnötige Reizung der Haut durch Reibungswärme entstehen kann. Auch Tierhaare zum Beispiel bei einem Hund können sich so nicht verfangen.
Das gesamte kleine Getriebe selbst mit Zahnrädern und Achsen kann ebenso aus Metall oder Kunststoff bestehen.
Der Motor und das Getriebe befinden sich im vorderen Teil des Gehäuses. Die Aufnahmevorrichtung befindet sich gut bedienbar außerhalb des Gehäuses.

**4.** Gehäuse mit Batterie und Verkabelung:
Das Gehäuse des *Tick-Rotator's* besteht aus Metall oder Kunststoff und sichert eine stabile Aufnahme des Motors mit seinem Getriebe, der Elektrik und der Energieträger im Inneren.
Es hat eine handliche, griffsichere und rutschfeste zylindrische Form.
Im Betrieb mit der Rotation hält man den Tick-Rotator wie eine Pinzette Daumen und Zeigefinger einfach über die Zecke.
Am Ende hat er ein kleines Gewinde auf das der Gehäusedeckel mit Ein- und Ausschalter gedreht wird.
Der Energieträger ist eine handelsübliche Batterie oder ein Akku.

**5.** Gehäusedeckel mit Schalter
Auch der Gehäusedeckel besteht aus dem Material oder Kunststoff wie das Gehäuse und hat ein kleines Gegengewinde um ihn auf das Gehäuse zu schrauben.
Im Deckel befindet sich ein Druckschalter zum Ein- und Ausschalten des *Tick-Rotator's* und der elektrische Pol mit Druckfeder für die Batterie.

**6.** Zentrierhülse:
Die durchsichtige Zentrierhülse aus Kunststoff oder Glas befindet sich abnehmbar an der vorderen Spitze des *Tick-Rotator's.* Sie ist zylindrisch vorne am Gehäuse aufgesteckt und hat zwei wesentliche Aufgaben.
Erstens ist sie eine Schutzhülle für den Pinselaufsatz, um einen unnötigen Verschleiß oder Verbiegungen des Pinsels zu vermeiden.
Wichtiger ist aber, dass der Benutzer durch den Aufsatz der Hülse auf der Haut um die Zecke herum einen rutschfesten, sicheren und auch genau zentrierten, mit der Zecke in der Mitte des Hülsenkreises,
Gebrauch durchführen kann. Durch das genaue Aufsetzen der durchsichtigen Zentrierhülse mit der Zecke in der Mitte sitzt der *Tick-Rotator* mit seiner Kegelaussparung im Pinsel genau auf der Zecke.
Nun kann mit der Rotation begonnen werden.
Die Zentrierhülse hat einen Durchmesser von ca. 10 bis 15mm.
Vorne schließt die Zentrierhülse genau mit dem Ende der Randpinselhaare ab, sodass sowohl Pinsel als auch Hülse gleichzeitig die Haut berühren.
Außerdem verhindert die Hülse, dass die Zecke nach dem Loslassen nicht herunterfällt.
Die Hülse ist jedoch für geübte Benutzer nicht zwingend notwendig.
Ebenso kann der *Tick-Rotator* auch bei der Benutzung an schwer zugänglichen Körperstellen ohne Zentrierhülse angewendet werden.
Anmerkung:
Einige Angaben und Abmessungen können sich noch geringfügig ändern, da sich der Prototyp noch in der Optimierungsphase befindet.
Alle geringfügigen Veränderungen oder Ergänzungen zu meiner jetzigen Patentanmeldung werde ich ihnen nachreichen.
Mein *Tick-Rotator* funktioniert zwar bisher mit vollen Erfolg, jedoch gibt es aufgrund der Filigranarbeiten und Beobachtungen der verschiedenen Zecken in den Pinselhaaren immer noch Feinabstimmungen zur Verbesserung der Funktion.

## Geänderte Patentansprüche

### Geänderte Patentansprüche gemäss Regel 137(2) EPÜ.

1. Vorrichtung zur Entfernung von Zecken aus der Haut eines Menschen oder eines Tieres
**gekennzeichnet durch**
ein Gehäuse mit einem an diesem ausgebildeten Griff;
einen drehbaren Pinselaufsatz; und
eine Aufnahmevorrichtung zur Aufnahme des Pinselaufsatzes;
wobei der Pinselaufsatz eine Haaranordnung mit Pinselhaaren aufweist, die aus einer Gruppe ausgewählt sind, welche Gruppe Kunsthaare und Naturhaare aufweist, und
wobei die Haaranordnung des Pinselaufsatzes eine mittig vorgesehene Vertiefung für das Platzieren eines Zeckenkörpers ausbildet.

2. Vorrichtung nach Anspruch 1, wobei ein elektrischer Antrieb für das Drehen des Pinselaufsatzes vorgesehen ist.

3. Vorrichtung nach einem der Ansprüche 1 und 2, ferner mit einem Elektromotor, der eine Antriebswelle aufweist, und mit einem Getriebe, wobei der Elektromotor gestaltet ist, um das Getriebe mit seiner Antriebswelle anzutreiben.

4. Vorrichtung nach Anspruch 3, wobei der Elektromotor und das Getriebe so gestaltet sind, dass ausgangsseitig des Getriebes eine Drehzahl von 60 bis 150 Umdrehungen pro Minute erzeugbar ist.

5. Vorrichtung nach einem der Ansprüche 3 und 4, wobei der Elektromotor und das Getriebe in dem Gehäuse angeordnet sind.

6. Vorrichtung nach einem der vorangehenden Ansprüche, wobei die Aufnahmevorrichtung außerhalb des Gehäuses angeordnet ist.

7. Vorrichtung nach einem der vorangehenden Ansprüche, wobei die Aufnahmevorrichtung zur Aufnahme des Pinselaufsatzes eine Aufnahmevorrichtung zur lösbaren Aufnahme des Pinselaufsatzes ist.

8. Vorrichtung nach einem der vorangehenden Ansprüche, wobei ein Gehäusedeckel vorgesehen ist, der als Schraubdeckel gestaltet ist und auf das Gehäuse schraubbar ist.

9. Vorrichtung nach Anspruch 7, wobei der Gehäusedeckel mit einem Ein- und Ausschalter versehen ist.

10. Vorrichtung nach einem der vorangehenden Ansprüche, wobei eine Batterie oder ein Akku als Energieträger vorgesehen und in dem Gehäuse angeordnet ist.

11. Vorrichtung nach einem der vorangehenden Ansprüche, wobei eine durchsichtige Zentrierhülse aus Kunststoff oder Glas abnehmbar am Gehäuse aufgesteckt ist, wobei die Zentrierhülse mit dem freien Ende der Randpinselhaare abschließt.

12. Vorrichtung nach einem der vorangehenden Ansprüche, wobei der Pinselaufsatz für seine Fixierung in der Aufnahmevorrichtung rückseitig einen Aufnahmestift aus Metall oder Kunststoff aufweist.

13. Pinselaufsatz für eine Vorrichtung gemäß einem der vorangehenden Ansprüche, wobei der Pinselaufsatz eine Haaranordnung mit Pinselhaaren aufweist, die aus einer Gruppe ausgewählt sind, welche Gruppe Kunsthaare und Naturhaare aufweist, und wobei die Haaranordnung des Pinselaufsatzes eine mittig vorgesehene Vertiefung für das Platzieren eines Zeckenkörpers ausbildet.

14. Verfahren zum Lösen einer in einer menschlichen oder tierischen Haut verankerten Zecke aus der Verankerungsstellung, wobei die Zecke, die die Fähigkeit hat zu krabbeln, bei diesem Lösen derart unversehrt bleibt, dass sie nach dem Lösen krabbeln kann, wobei, insbesondere mit einer Vorrichtung gemäß einem der vorangehenden Ansprüche, ein Pinselaufsatz, der eine Haaranordnung mit Pinselhaaren aufweist, die mittig eine Vertiefung ausbildet, mit seiner Vertiefung auf die Zecke aufgesetzt und anschließend gedreht wird, so dass die Zecke aus Ihrer Verankerung in der Haut gelöst wird.

15. Verfahren nach Anspruch 14, wobei das Drehen des Pinselaufsatzes gleichmäßig und in mehreren Intervallen erfolgt.
